# EUROPEAN PATENT APPLICATION

(11) **EP 4 752 207 A1**
(43) Date of publication of application: **03.06.2026**
(21) Application number: 24216453.1
(22) Date of filing: 29.11.2024
(51) Int. Cl.: C12M 1/00, C12M 1/06, C12M 1/36

(54) **METHOD OF TRANSFERRING A MICROBIAL BIOPROCESS FROM A MULTI-USE BIOREACTOR TO A SINGLE-USE BIOREACTOR**

(71) Applicant: Sartorius Stedim Biotech GmbH, 37079 Göttingen (DE)
(72) Inventor: Leupold, Marco, 34302 Guxhagen (DE); Rupprecht, Jens, 37079 Göttingen (DE)
(74) Representative: Gottschald Patentanwälte Partnerschaft mbB

(57) **Abstract**

The invention relates to a method of transferring a microbial bioprocess from a multi-use bioreactor (1) to a single-use bioreactor (2), wherein the source bioprocess is a batch or fed-batch microbial bioprocess and the source bioprocess arrangement (3) comprises a source bioreactor (5) with a stirrer and the source bioreactor (5) is the multi-use bioreactor (1) which has at least 1000 L production volume of a microbial culture producing a maximum heat of at least 25,000 W and/or the stirrer of the source bioreactor (5) is driven with a maximum torque of at least 120 Nm, wherein the target bioprocess is a continuous microbial bioprocess and the target bioprocess arrangement (4) comprises a target bioreactor (6) and the target bioreactor (6) is the single-use bioreactor (2) which has a production volume of a microbial culture of at most 50 % of the production volume of the source bioreactor (5) and of at most 800 L, the microbial culture of the target bioreactor (6) produces a maximum heat of at most 20,000 W during the target bioprocess and/or the target bioreactor (6) comprises a stirrer driven with a maximum torque of at most 100 Nm, wherein an average amount of the bioproduct produced per time of performance of the target bioprocess by the target bioreactor (6) is at least 90 %, preferably at least 100 %, of an average amount of the bioproduct produced per time of performance of the source bioprocess by the source bioreactor (5).

## Description

The present invention relates to a method of transferring a microbial bioprocess from a multi-use bioreactor to a single-use bioreactor according to claim 1 to a method of performing the target bioprocess according to claim 12 and to a computer system for supporting a transfer of a microbial bioprocess from a multi-use bioreactor to a single-use bioreactor according to claim 13.

Bioprocesses can be roughly separated into bioprocesses based on mammalian cells (e.g., CHO cells), and microbial bioprocesses, based on for example bacteria (e.g., *E. coli*)*,* yeast (e.g., *Pichia pastoris*) or fungi (e.g., *Trichoderma* reesei). Microbial bioprocesses have different challenges than mammalian bioprocesses, and are often more demanding regarding the transfer of oxygen, cooling, foam regulation and feed regulation. Many large scale microbial bioprocesses are currently performed in stainless steel, multi-use bioreactors as batch or fed-batch processes. A stainless steel bioreactor typically allows high oxygen transfer rates and provides high cooling power and is overall a very powerful system that suits the high demands of microbial bioprocesses. However, such a process faces challenges regarding the time and validation needed for cleaning with resulting contamination risks, has a low flexibility for changes, a high capital and space investment, high maintenance costs and a high effort for the overall facility design regarding media tanks and the like.

The invention is based on the problem of improving the known methods such that a further optimization regarding the named challenges is reached.

The above-noted object is solved by the features of claim 1.

The first realization underlying the present invention is that bioprocesses based on single-use bioreactors can generally achieve an improvement for all the named challenges. However, due to the larger capabilities and high process volumes of stainless steel bioreactors, some bioprocesses running in stainless steel bioreactors with high oxygen uptake rates translating into high process heat and therefore high cooling requirements and high stirrer torque needed to transfer the oxygen, cannot be translated satisfactorily into single-use bioreactors. As the temperature within the bioreactor is normally controlled and kept at a certain value (e.g., at 37°C), the heat generated needs to be removed to maintain this temperature. Heat removal in stainless steel bioreactors is often achieved by providing cooling water in a cooling jacket. Exemplarily, a bioprocess using an already low cooling water temperature of 5° C in a stainless steel bioreactor would require sub-zero cooling temperatures in a usual single-use bioreactor, where the heat transfer is less efficient. Additionally, the torque needed to stir such a bioreactor may be so high that a single-use magnetically driven stirrer cannot be used and/or introduces too much additional heat. Very highly specialized single-use equipment may be a solution in some cases, however this may increase costs to a level not acceptable. Translating such a bioprocess into a single-use bioreactor is therefore not feasible. At least in part due to the reasons mentioned above, the production volume of a single stainless steel bioreactor is currently not yet reached by a single-use bioreactor.

The main realization of the present invention is that at least some of the advantages of single-use equipment can still be realized for a process which uses process volumes with process parameters that cannot be used on regular single-use bioreactors, here high energy uptake/heat production and stirring torque, which have been identified as main limitations for a transfer to single-use bioreactors, can still be transferred to single-use bioreactors without losing a relevant amount of produced product per time by changing the process mode from batch or fed-batch to a continuous process mode.

A continuous process, in particular a continuous process run as a chemostat, has two main advantages leading to a higher productivity (product per volume and time). A continuous process can get closer to the limit of the limiting process parameters, primarily maximum produced heat allowed and maximum stirrer torque allowed, and stay close to the limit for a longer time, than a batch or fed-batch process which normally has an increasing heat generation until reaching the limit and then being stopped. Further, a continuous process can be run for a longer time, decreasing the percentage of time in which the process is not running due to changeovers (cleaning, etc.). The use of single-use components increases the latter advantage further but decreases the limits of the limiting process parameters as discussed above. Nevertheless, the increased productivity allows reducing the process volume and thereby reducing, in particular the limiting, process parameters into a range compatible with single-use bioreactors. Other factors that can also contribute to a higher productivity in a continuous process include better conditions for the microorganisms due to steady-state conditions, a lower product concentration in the culture medium, and the possibility to keep the cells at a certain growth rate.

The present realization therefore hinges on two steps. First, the realization that single-use components would be advantageous but cannot be used with a given process and second, the realization that the process mode can be changed to increase the mean productivity (product per time and per volume averaged over a long time) in particular by increasing the time in which the process runs near its limits (i.e. with a high productivity) and at the same time reduce the process volume while keeping the overall output (productivity x volume) at least mostly constant. Therefore, single-use components can be used, and their advantages can be achieved. An additional benefit of employing single-use systems in combination with continuous process execution is the achievable significant reduction of consumables costs compared to fed-batch processes. Compared to stainless-steel fermenter the overall cost and footprint reduction would provide an additional benefit.

The use of single-use bioreactors then leads to a number of advantages. The risk of contamination is decreased, the need for cleaning and cleaning validation is decreased or even completely removed, a faster product change-over is possible, the capital investment may be reduced, for example if a second plant is newly set-up compared to using the same process as before, the utility requirements (sterilization, cleaning, piping) can be reduced, the flexibility in the facility design is increased, a quicker implementation can be achieved, and the maintenance effort is decreased. Also, with a smaller bioreactor production volume the pre-culture effort for a starting the microbial bioprocess may be reduced. As will be explained, not all the advantages are necessarily achieved, but forgoing some of the advantages may even lead to other advantages.

In detail, a method of transferring a microbial bioprocess from a multi-use bioreactor to a single-use bioreactor, wherein the method comprises, based on source specification data of a source bioprocess performed on a source bioprocess arrangement, generating target specification data of a target bioprocess performed on a target bioprocess arrangement, wherein, according to the source specification data, the source bioprocess is a batch or fed-batch microbial bioprocess and the source bioprocess arrangement comprises a source bioreactor with a stirrer and the source bioreactor is the multi-use bioreactor which has at least 1,000 L production volume of a microbial culture comprising a type of microorganism producing a bioproduct and the microbial culture of the source bioreactor produces a maximum heat of at least 25,000 W during the source bioprocess and/or the stirrer of the source bioreactor is driven with a maximum torque of at least 120 Nm, wherein, according to the target specification data, the target bioprocess is a continuous microbial bioprocess and the target bioprocess arrangement comprises a target bioreactor and the target bioreactor is the single-use bioreactor which has a production volume of a microbial culture of at most 50% of the production volume of the source bioreactor and of at most 800 L, the microbial culture of the target bioreactor produces a maximum heat of at most 20,000 W during the target bioprocess and/or the target bioreactor comprises a stirrer driven with a maximum torque of at most 100 Nm, wherein an average amount of the bioproduct produced per time of performance of the target bioprocess by the target bioreactor is at least 90%, preferably at least 100%, of an average amount of the bioproduct produced per time of performance of the source bioprocess by the source bioreactor, is proposed.

The method may be partially of fully computer-implemented, in particular the generation of the target specification data may be done by a computer system. Claim 2 refers to embodiments describing a suitable computer system.

The method may also comprise the implementation of the target bioprocess according to an embodiment described in claim 3.

In embodiments according to claim 4, the target bioprocess arrangement comprises at least one double-use component, which was already part of the source bioprocess arrangement, allowing to implement the target bioprocess with reduced cost. Additionally, the double-use component's performance and functionality are already known and validated. In these embodiments, the method comprises re-using part of the source bioprocess arrangement and therefore re-equipping the source bioprocess arrangement into the target bioprocess arrangement. It is then the case that the source bioprocess performed on the existing source bioprocess arrangement is transferred to the target bioprocess at the same location and by using the target bioreactor instead of the source bioreactor.

For example, a possible double-use component, according to claim 5, may be a control unit which may already be programmed for the source bioprocess and is then adapted for the target bioprocess. Reusing part of a recipe and therefore a part of the setpoints for certain process parameters makes the transfer less complex.

It may even be the case that the target bioprocess arrangement differs significantly from a usual continuous single-use bioprocess arrangement due to the double-use component or components. In an embodiment according to claim 6 it is proposed to use a container, for example a tank for a feed substrate, piping, cooling equipment, downstream or pre-culture components of the source bioprocess arrangement in the target bioprocess arrangement. In single-use based bioprocess arrangements, usually, the culture medium is supplied pre-sterilized (e.g., by sterile-filtration) from a single-use media tank while multi-use based bioprocess arrangements regularly use media tanks where the culture medium is sterilized (e.g., by steam-sterilization) in place. However, if media tanks are already present, re-using these in a single-use bioprocess can be more efficient. The same is true for other components, resulting in a patch-work type target bioprocess arrangement.

Claim 7 concerns preferred microorganisms. Claim 8 concerns a preferred mean growth rate which is preferably higher in the target bioprocess, showing how an increased productivity can be achieved in a continuous process.

Another embodiment contributing to the increased productivity according to claim 9 relates to a fewer number of restarts per time in the continuous process. A batch and/or fed-batch usually process ends at the latest, when a limit of a limiting process parameter, e.g. the maximum possible heat removal capacity, of the bioreactor is reached. This limit is therefore made use of for only a moment. In a continuous process, the process parameter can be continuously at or near the limit and there is no need to stop the process based on reaching this limit.

Claim 10 relates to preferred values for the bioreactor volumes and limiting process parameters.

Claim 11 describes the target bioprocess.

Another teaching according to claim 12, which is of equal importance, relates to a method of performing the target bioprocess according to the target specification data derived by the proposed method.

All explanations given with regard to the proposed method are fully applicable.

Another teaching according to claim 13, which is of equal importance, relates to a computer system for supporting a transfer of a microbial bioprocess from a multi-use bioreactor to a single-use bioreactor, wherein the computer system comprises an input data interface and an output data interface, wherein the computer system comprises a processor and a non-volatile memory, wherein the computer system comprises computer instructions stored in the non-volatile memory that cause the system to perform a method of transferring a microbial bioprocess from a multi-use bioreactor to a single-use bioreactor, wherein the method comprises, based on source specification data of a source bioprocess performed on a source bioprocess arrangement, generating target specification data of a target bioprocess performed on a target bioprocess arrangement, wherein, according to the source specification data, the source bioprocess is a batch or fed-batch microbial bioprocess and the source bioprocess arrangement comprises a source bioreactor with a stirrer and the source bioreactor is a multi-use bioreactor with at least 1000 L production volume of a microbial culture comprising a type of microorganism producing a bioproduct and the microbial culture of the source bioreactor produces a maximum heat of at least 25,000 W during the source bioprocess and/or the stirrer of the source bioreactor is driven with a maximum torque of at least 120 Nm, wherein, according to the target specification data, the target bioprocess is a continuous microbial bioprocess and the target bioprocess arrangement comprises a target bioreactor and the target bioreactor is a single-use bioreactor with a production volume of a microbial culture of at most 50% of the production volume of the source bioreactor and of at most 800 L, the microbial culture of the target bioreactor produces a maximum heat of at most 20,000 W during the target bioprocess and/or the target bioreactor comprises a stirrer driven with a maximum torque of at most 100 Nm, wherein an average amount of the bioproduct produced per time of performance of the target bioprocess by the target bioreactor is at least 90%, preferably at least 100%, of an average amount of the bioproduct produced per time of performance of the source bioprocess by the source bioreactor.

All explanations given with regard to the proposed methods are fully applicable. The computer system may comprise computer instructions stored in the memory that cause the system to perform any of the steps described with regard to the proposed method as far as these are executable by the computer system.

In the following, embodiments of the invention are explained with respect to the drawing. The drawing shows in
- Fig. 1: a source bioprocess arrangement,
- Fig. 2: a target bioprocess arrangement and
- Fig. 3: a schematic comparison of heat (or similarly other limiting process parameters) between a fed-batch bioprocess and a continuous bioprocess.

In the following, continuous and non-continuous bioprocesses are described. It is generally known which bioprocesses are continuous and which are not. A repeated fed-batch is here seen as a continuous bioprocess. The following describe differences between the different types of bioprocess, should however not serve as complete definitions or be understood as limiting the common understanding of the different types of bioprocesses. A main difference between continuous and non-continuous bioprocesses is the timing of the harvest (intermediate or continuous vs. at the end).

Batch Process: A closed system where microorganisms, nutrients, and media are added at the start, and the process runs to completion without further additions or removals. Harvest occurs at the end.

Fed-Batch Process: An extension of batch processing where nutrients are added continuously or intermittently to the bioreactor while the microorganisms grow and produce product. Harvest occurs at the end.

Repeated Fed-Batch Process: A variation of fed-batch where a portion of the culture is removed and fresh medium is added after each cycle, but not continuously. This process is repeated multiple times.

Continuous Process (perfusion or chemostat): An open system where fresh medium is continuously added, and spent medium and product are continuously removed, maintaining a steady-state culture. In a chemostat process, microorganisms are removed with the spent medium and product while in a perfusion process the microorganisms are retained by using a filter. An intermediate form in which only a small amount of microorganisms are removed and others are retained via a filter or any other form of modified continuous process is also conceivable.

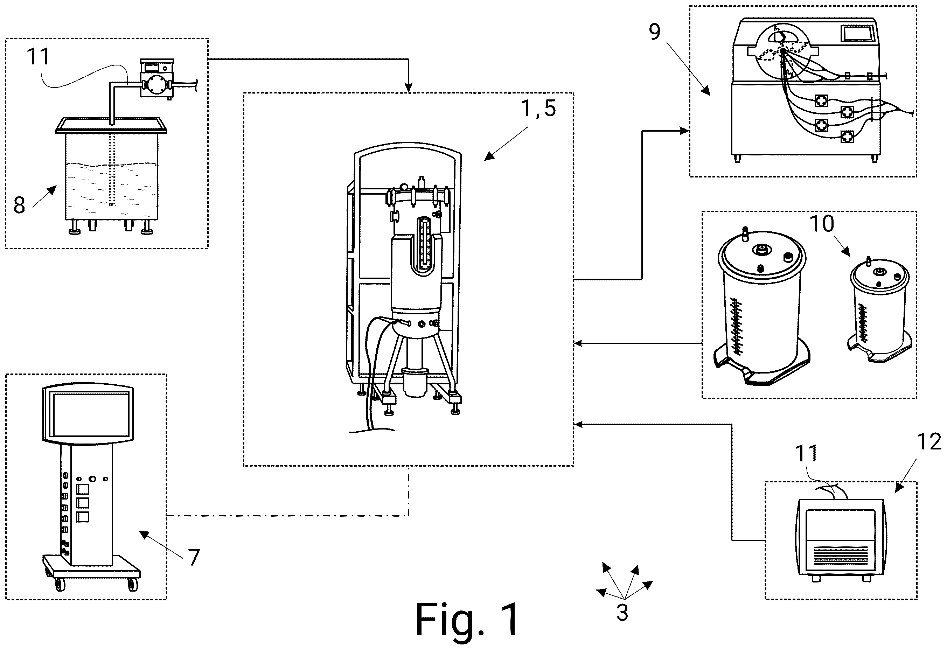

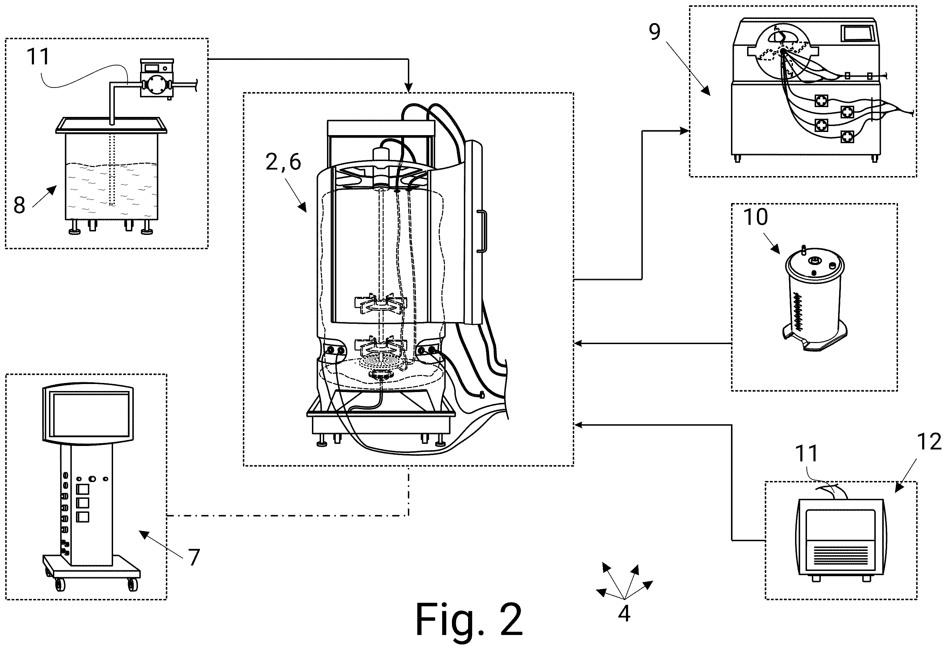

Proposed is a method of transferring a microbial bioprocess from a multi-use bioreactor 1 (Fig. 1) to a single-use bioreactor 2 (Fig. 2).

The method comprises, based on source specification data of a source bioprocess performed on a source bioprocess arrangement 3, generating target specification data of a target bioprocess performed on a target bioprocess arrangement 4. As a starting point for the method there is therefore a source bioprocess that is already used in practice. The method therefore does not relate to the development of a new bioprocess but to the improvement of an existing bioprocess. The specification of this existing bioprocess exists in any form of source specification data. From this source specification data, a specification for a derived bioprocess, the target bioprocess, is derived. The source bioprocess is performed with a multi-use bioreactor 1 while the target bioprocess is performed with a single-use bioreactor 2. The main target of the transfer is therefore to change the source bioprocess such that it can be performed in a single-use bioreactor 2. Consequently, the source bioprocess is a bioprocess that cannot be performed in a single-use bioreactor 2 at least without considerable challenges regarding performance and/or costs.

According to the source specification data and consequently preferably in at least one execution of the source bioprocess in real, the source bioprocess is a batch or fed-batch microbial bioprocess. The source bioprocess is therefore not continuous and the general field of the proposed solution is that of microorganism-based bioprocesses.

Further, according to the source specification data and consequently preferably in at least one execution of the source bioprocess in real, the source bioprocess arrangement 3 comprises a source bioreactor 5 with a stirrer and the source bioreactor 5 is the multi-use bioreactor 1. Fig. 1 shows an exemplary source bioprocess arrangement 3 with such a multi-use bioreactor 1, which here and preferably is a stainless steel bioreactor. The stirrer of the source bioreactor 5 may comprise a magnetic drive.

Still according to the source specification data and consequently preferably in at least one execution of the source bioprocess in real, the multi-use bioreactor 1 has at least 1000 L production volume of a microbial culture comprising a type of microorganism producing a bioproduct. The production volume (also called working volume) is the volume that is actually usable for the microbial culture.

It is further the case, according to the source specification data and consequently preferably in at least one execution of the source bioprocess in real, that the microbial culture of the source bioreactor 5 produces a maximum heat of at least 25,000 W during the source bioprocess and/or the stirrer of the source bioreactor 5 is driven with a maximum torque of at least 120 Nm. Both parameters are seen as being above a soft boundary for a transfer of the source bioprocess to a single-use bioreactor 2. Providing cooling of 25,000 W is hard to impossible with usual single-use bioreactors 2 of any size. Single-use materials often provide a higher insulation themselves and especially non-rigid bags also have a decreased surface contact with a cooling mantle or the like. While special kinds of single-use materials may be conceivable, the lack of commercial availability at decent prices defeats many of the main purposes of single-use. A driving torque of 120 Nm is indirectly linked to the oxygen consumption, if the process is oxygen-based, on the one hand and on the other hand usual magnetically driven stirrers may not be able to support such a high torque. Therefore, the source bioprocess cannot be transferred to a single-use bioreactor 2 without problems.

The proposed solution is that, according to the target specification data and consequently preferably in at least one execution of the target bioprocess in real, the target bioprocess is a continuous microbial bioprocess. Leveraging the increased productivity of a continuous bioprocess allows adapting a single-use bioreactor 2. Therefore, according to the target specification data and consequently preferably in at least one execution of the target bioprocess in real, the target bioprocess arrangement 4 comprises a target bioreactor 6 and the target bioreactor 6 is the single-use bioreactor 2. It is further the case that, according to the target specification data and consequently preferably in at least one execution of the target bioprocess in real, the single-use bioreactor 2 has a production volume of a microbial culture of at most 50 % of the production volume of the source bioreactor 5 and of at most 800 L. Depending on the volume of the source bioreactor 5, the target bioreactor 6 is significantly smaller.

It is then the case that, according to the target specification data and consequently preferably in at least one execution of the target bioprocess in real, the microbial culture of the target bioreactor 6 produces a maximum heat of at most 20,000 W during the target bioprocess and/or the target bioreactor 6 comprises a stirrer driven with a maximum torque of at most 100 Nm. Conceivably, the target bioreactor 6 may comprise a different means for mixing than a stirrer. However, if the target bioreactor 6 comprises a stirrer, it is preferably a stirrer that comprises a magnetic drive. As can be seen, the limiting process parameters are reduced. Fig. 2 shows an exemplary target bioprocess arrangement 4 with a single-use bioreactor 2. Naturally, after each execution of the target bioprocess, the single-use bioreactor 2 is exchanged for a new single-use bioreactor 2.

Nevertheless, an average amount of the bioproduct produced per time of performance of the target bioprocess by the target bioreactor 6 is at least 90 %, preferably at least 100 %, of an average amount of the bioproduct produced per time of performance of the source bioprocess by the source bioreactor 5.

This is achieved by leveraging the higher productivity of the continuous process and preferably by also increasing the growth rate of the microorganisms, as will be explained. Here, the average amount of bioproduct produced per time is looked at as in the end, an existing bioprocess should not be transferred into a bioprocess with significantly less overall yield. It is also less relevant what amount of bioproduct is produced per volume or at a certain point in time, as in the end, an absolute mass is sold. The average amount is therefore calculated by taking into account a long enough time, for example one execution of the respective bioprocess and the time needed to clean and replace parts until the next execution can be started. The production volume is not taken into account, i.e. if the source bioprocess produced 1 kg of product per week, the target bioprocess must produce at least 900 g of product per week.

It is also evident that a continuous process can be run for a longer time until stopping its execution such that the reduced time for replacing parts and the like and the reduced number of restarts both increase the average amount of product produced. Setup times are explicitly included in the calculation.

It is preferably the case that a computer system is provided, which computer system comprises an input data interface and an output data interface, and, that the method comprises receiving, via the input data interface, the source specification data and outputting, via the output data interface, the target specification data. The computer system may comprise a processor and/or a, preferably non-volatile, memory. The computer system may be a cloud computer system with distributed computing or the like. The input data interface may comprise a website for receiving the source specification data and/or the output data interface may comprise a website for outputting the target specification data.

According to one embodiment the method comprises executing the target bioprocess by the target bioprocess arrangement 4. The method may also comprise the last execution of the source bioprocess at the source bioprocess arrangement 3.

It is possible that the target bioprocess is performed at the same location as the source bioprocess and that the source bioprocess arrangement 3 is disassembled when or prior to assembling the target bioprocess arrangement 4. It may be the case that the target bioprocess arrangement 4 comprises at least one double-use component of the source bioprocess arrangement 3, which double-use component was used for the source bioprocess, in particular connected to the source bioreactor 5, and is used for the target bioprocess, in particular connected to the target bioreactor 6. It may therefore be part of the proposed method that the source bioprocess arrangement 3 is changed into the target bioprocess arrangement 4 and the double-use component is kept.

This embodiment is particularly interesting in several cases and allows reducing the cost for setting up the target bioprocess arrangement 4.

In one embodiment the at least one double-use component comprises a control unit 7 for controlling the source bioreactor 5 and subsequently the target bioreactor 6. Both figures show the control unit 7, which is here reused.

Alternatively or additionally, the method may comprise generating, from a source recipe used, in particular by the control unit 7, to control the source bioreactor 5, a target recipe and using, in particular by the control unit 7, the target recipe to control the target bioreactor 6. The generating may be performed by the computer system or the control unit 7. The computer system may receive the source recipe via the input data interface and preferably automatically extracts at least some, preferably all, of the source specification data from the source recipe. Preferably, the computer system is connected to or comprises the control unit 7.

In another case or additionally, the at least one double-use component may comprise a container 8, in particular multi-use tank, for a process medium, in particular feed substrate, and/or one or more downstream components 9 and/or one or more pre-culture components 10 and/or piping 11 and/or cooling equipment 12.

Depending on the existing equipment it may be the case that pre-culture components 10 like a smaller, possibly multi-use, bioreactor or downstream components 9 are re-used as double-use components. It may also be the case that a series of connected downstream components 9 is re-used.

It is particularly interesting however that also components not usually associated with single-use bioreactors 2 like piping 11, in particular leading to the bioreactor, and/or the container 8 for the process medium may be re-used. Usually for single-use bioreactors 2, pre-sterilized process media in single-use bags are used which may have been sterilized by sterile filtration. Once emptied, these single-use bags are not reused, but exchanged by a new single-use bag. The process medium may be provided in a multi-use container in which at least a part of the process medium is sterilized within the multi-use container by steam sterilization.

According to one embodiment it is proposed, that, according to the source specification data and the target specification data, the microorganism is a bacterium, in particular *E. coli,* or, that the microorganism is a yeast, in particular *Pichia pastoris.* It is preferably the case, that the microorganism has a positively growth-coupled productivity. A smaller bioreactor with a higher growth rate is increasingly useful if the productivity is positively growth-coupled, i.e. increases with the growth rate. It may be the case that the microorganism has a superlinearly positively growth-coupled productivity, i.e. the productivity increases more than the growth, for example polynomially.

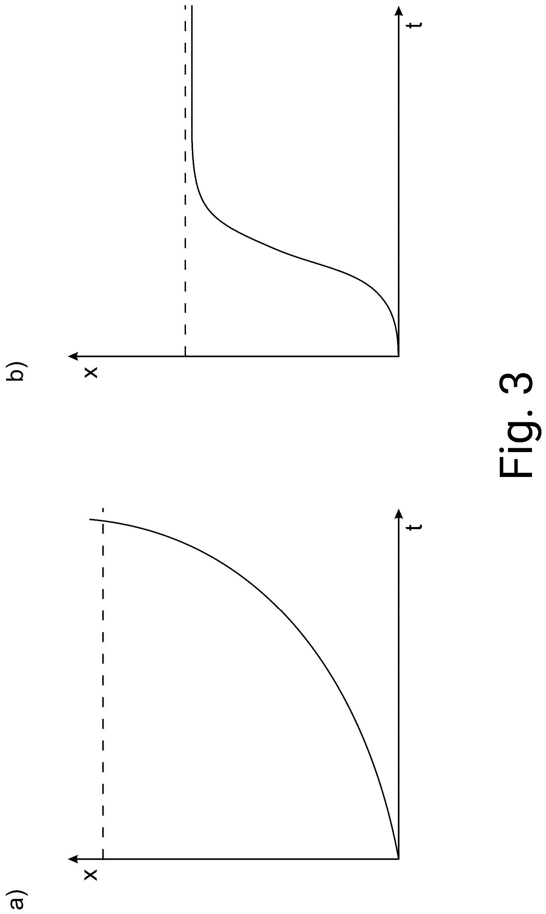

As already mentioned, it is preferably the case that, according to the source specification data and the target specification data, and consequently preferably in at least one execution of the source bioprocess and/or the target bioprocess in real, a mean growth rate of the microorganism in a steady state of the target bioprocess is higher than a mean growth rate of the microorganism during a fed-batch phase in the source bioprocess. Increasing the growth rate is one factor to increase the productivity for the continuous bioprocess. In a fed-batch process the growth rate may be set and held approximately constant by an exponential feed. During feeding, the culture volume will increase. In a batch process, the comparison of mean growth rates is less meaningful. In a continuous process, the growth rate corresponds to the dilution rate and can therefore be kept constant by keeping the dilution rate constant (chemostat). Depending on the process conditions (e.g., the dilution rate set, the carbon source concentration in the feed medium etc.), a continuous cultivation will require some time before reaching steady-state conditions. Once the steady-state has been reached, the parameters of the process, in particular the culture volume, is kept constant. Fig. 3 shows an increase in biomass in a fed-batch process (fig. 3 a)) and a continuous process (fig. 3 b)). "x" may stand for the biomass, however also symbolizes the limiting process parameter, in particular heat generation and/or oxygen consumption, which is/are dependent on the biomass and exhibit the same behavior. The dashed line indicates a maximum of said process parameter, here maximum heat removal capacity and/or maximum oxygen supply. It is smaller in the continuous single-use bioreactor 2 in fig. 3 b). However, as can be seen, the growth rate in fig. 3 b) can be higher at the beginning and can then be kept constant without hitting the limit while in the fed-batch process the process parameter is far from the limit most of the time and then hits the limit with exponential growth.

The continuous process may therefore have a greater area-under-the-curve as the limit is exploited more efficiently. Additionally, the continuous process may run for a longer time, reducing the losses from set-up times. A further effect playing into the efficiency of the continuous process is that the conditions for the microorganisms can be set better, for example by reducing the concentration of the product, which may also increase productivity as a product inhibition may be prevented. These effects together allow better utilizing the available limit of the process parameters, even if these limits are lower with single-use bioreactors 2 than with multi-use bioreactors 1.

Therefore, according to one embodiment it is proposed, that, according to the source specification data and the target specification data, and consequently preferably in at least one execution of the source bioprocess and/or the target bioprocess in real, a number of restarts per time of the target bioprocess is smaller than a number of restarts per time of the source bioprocess. Preferably, according to the source specification data and the target specification data, and consequently preferably in at least one execution of the source bioprocess and/or the target bioprocess in real, the number of restarts per time of the target bioprocess is at most half, preferably at most a quarter, of the number of restarts per time of the source bioprocess.

According to one embodiment it is proposed, that, according to the source specification data, and consequently preferably in at least one execution of the source bioprocess in real, the production volume of the source bioreactor 5 is at least 2,000 L, preferably at least 3,000 L, more preferably at least 4,000 L, and/or at most 10,000 L and/or the microbial culture of the source bioreactor 5 produces a maximum heat of at least 30,000 W, preferably at least 35,000 W, and/or at least 30 W/L, preferably at least 40 W/L, during the source bioprocess, and/or the stirrer of the source bioreactor 5 is driven with a maximum torque of at least 140 Nm, preferably at least 180 Nm.

Additionally or alternatively, according to the target specification data, and consequently preferably in at least one execution of the target bioprocess in real, the production volume of the target bioreactor 6 is at most 600 L, preferably at most 500 L, more preferably at most 300 L, and/or at least 10 L, preferably at least 100L, and/or the microbial culture of the target bioreactor 6 produces a maximum heat of at most 18,000 W, preferably at most 15,000 W, more preferably at most 10,000 W, and/or at most 80%, preferably at most 60%, of the maximum heat produced by the source bioreactor 5 according to the source specification data, and/or the target bioreactor 6 comprises a stirrer driven with a maximum torque of at most 80 Nm, preferably at most 60 Nm.

It is preferably the case that, according to the target specification data, and consequently preferably in at least one execution of the target bioprocess in real, the target bioprocess is a chemostat or perfusion process.

Another teaching which is of equal importance relates to a method of performing the target bioprocess according to the target specification data derived by the proposed method.

Another teaching which is of equal importance relates to a computer system for supporting a transfer of a microbial bioprocess from a multi-use bioreactor 1 to a single-use bioreactor 2, wherein the computer system comprises an input data interface and an output data interface, wherein the computer system comprises a processor and a non-volatile memory, wherein the computer system comprises computer instructions stored in the non-volatile memory that cause the system to perform a method of transferring a microbial bioprocess from a multi-use bioreactor 1 to a single-use bioreactor 2, wherein the method comprises, based on source specification data of a source bioprocess performed on a source bioprocess arrangement 3, generating target specification data of a target bioprocess performed on a target bioprocess arrangement 4, wherein, according to the source specification data, the source bioprocess is a batch or fed-batch microbial bioprocess and the source bioprocess arrangement 3 comprises a source bioreactor 5 with a stirrer and the source bioreactor 5 is a multi-use bioreactor 1 with at least 1000 L production volume of a microbial culture comprising a type of microorganism producing a bioproduct and the microbial culture of the source bioreactor 5 produces a maximum heat of at least 25,000 W during the source bioprocess and/or the stirrer of the source bioreactor 5 is driven with a maximum torque of at least 120 Nm, wherein, according to the target specification data, the target bioprocess is a continuous microbial bioprocess and the target bioprocess arrangement 4 comprises a target bioreactor 6 and the target bioreactor 6 is a single-use bioreactor 2 with a production volume of a microbial culture of at most 50 % of the production volume of the source bioreactor 5 and of at most 800 L, the microbial culture of the target bioreactor 6 produces a maximum heat of at most 20,000 W during the target bioprocess and/or the target bioreactor 6 comprises a stirrer driven with a maximum torque of at most 100 Nm, wherein an average amount of the bioproduct produced per time of performance of the target bioprocess by the target bioreactor 6 is at least 90 %, preferably at least 100 %, of an average amount of the bioproduct produced per time of performance of the source bioprocess by the source bioreactor 5.

## Claims

1. Method of transferring a microbial bioprocess from a multi-use bioreactor (1) to a single-use bioreactor (2), wherein the method comprises, based on source specification data of a source bioprocess performed on a source bioprocess arrangement (3), generating target specification data of a target bioprocess performed on a target bioprocess arrangement (4),
wherein, according to the source specification data,
the source bioprocess is a batch or fed-batch microbial bioprocess and
the source bioprocess arrangement (3) comprises a source bioreactor (5) with a stirrer and
the source bioreactor (5) is the multi-use bioreactor (1) which has at least 1,000 L production volume of a microbial culture comprising a type of microorganism producing a bioproduct and
the microbial culture of the source bioreactor (5) produces a maximum heat of at least 25,000 W during the source bioprocess and/or
the stirrer of the source bioreactor (5) is driven with a maximum torque of at least 120 Nm,
wherein, according to the target specification data,
the target bioprocess is a continuous microbial bioprocess and
the target bioprocess arrangement (4) comprises a target bioreactor (6) and the target bioreactor (6) is the single-use bioreactor (2) which has a production volume of a microbial culture of at most 50% of the production volume of the source bioreactor (5) and of at most 800 L,
the microbial culture of the target bioreactor (6) produces a maximum heat of at most 20,000 W during the target bioprocess and/or
the target bioreactor (6) comprises a stirrer driven with a maximum torque of at most 100 Nm,
wherein an average amount of the bioproduct produced per time of performance of the target bioprocess by the target bioreactor (6) is at least 90%, preferably at least 100%, of an average amount of the bioproduct produced per time of performance of the source bioprocess by the source bioreactor (5).

2. Method according to claim 1, **characterized in that** a computer system is provided, that the computer system comprises an input data interface and an output data interface, and, that the method comprises receiving, via the input data interface, the source specification data and outputting, via the output data interface, the target specification data.

3. Method according to claim 1 or 2, **characterized in that** the method comprises executing the target bioprocess by the target bioprocess arrangement (4).

4. Method according to one of the preceding claims, **characterized in that** the target bioprocess arrangement (4) comprises at least one double-use component of the source bioprocess arrangement (3), which double-use component was used for the source bioprocess, in particular connected to the source bioreactor (5), and is used for the target bioprocess, in particular connected to the target bioreactor (6).

5. Method according to claim 4, **characterized in that** the at least one double-use component comprises a control unit (7) for controlling the source bioreactor (5) and subsequently the target bioreactor (6), and/or, that the method comprises generating, from a source recipe used, in particular by the control unit (7), to control the source bioreactor (5), a target recipe and using, in particular by the control unit (7), the target recipe to control the target bioreactor (6).

6. Method according to claim 4 or 5, **characterized in that** the at least one double-use component comprises a container (8), in particular multi-use tank, for a process medium, in particular feed substrate, and/or one or more downstream components (9) and/or one or more pre-culture components (10) and/or piping (11) and/or cooling equipment (12).

7. Method according to one of the preceding claims, **characterized in that**, according to the source specification data and the target specification data, the microorganism is a bacterium, in particular E.coli, or, that the microorganism is a yeast, in particular Pichia pastoris, preferably, that the microorganism has a positively growth-coupled productivity.

8. Method according to one of the preceding claims, **characterized in that**, according to the source specification data and the target specification data, a mean growth rate of the microorganism in a steady state of the target bioprocess is higher than a mean growth rate of the microorganism in a fed-batch phase in the source bioprocess.

9. Method according to one of the preceding claims, **characterized in that**, according to the source specification data and the target specification data, a number of restarts per time of the target bioprocess is smaller than a number of restarts per time of the source bioprocess, preferably, that, according to the source specification data and the target specification data, the number of restarts per time of the target bioprocess is at most half, preferably at most a quarter, of the number of restarts per time of the source bioprocess.

10. Method according to one of the preceding claims, **characterized in that**, according to the source specification data, the production volume of the source bioreactor (5) is at least 2000 L, preferably at least 3000 L, more preferably at least 4000 L, and/or at most 10,000 L and/or the microbial culture of the source bioreactor (5) produces a maximum heat of at least 30,000 W, preferably at least 35,000 W, and/or at least 30 W/L, preferably at least 40 W/L, during the source bioprocess, and/or the stirrer of the source bioreactor (5) is driven with a maximum torque of at least 140 Nm, preferably at least 180 Nm, and/or, that according to the target specification data, the production volume of the target bioreactor (6) is at most 600 L, preferably at most 500 L, more preferably at most 300 L, and/or at least 10 L, preferably at least 100L, and/or the microbial culture of the target bioreactor (6) produces a maximum heat of at most 18,000 W, preferably at most 15,000 W, more preferably at most 10,000 W, and/or at most 80 %, preferably at most 60 %, of the maximum heat produced by the source bioreactor (5) according to the source specification data, and/or the target bioreactor (6) comprises a stirrer driven with a maximum torque of at most 80 Nm, preferably at most 60 Nm.

11. Method according to one of the preceding claims, **characterized in that**, according to the target specification data, the target bioprocess is a chemostat or perfusion process.

12. Method of performing the target bioprocess according to the target specification data derived by the method according to one of the preceding claims.

13. Computer system for supporting a transfer of a microbial bioprocess from a multi-use bioreactor (1) to a single-use bioreactor (2), wherein the computer system comprises an input data interface and an output data interface, wherein the computer system comprises a processor and a non-volatile memory, wherein the computer system comprises computer instructions stored in the non-volatile memory that cause the system to perform a method of transferring a microbial bioprocess from a multi-use bioreactor (1) to a single-use bioreactor (2), wherein the method comprises, based on source specification data of a source bioprocess performed on a source bioprocess arrangement (3), generating target specification data of a target bioprocess performed on a target bioprocess arrangement (4),
wherein, according to the source specification data,
the source bioprocess is a batch or fed-batch microbial bioprocess and
the source bioprocess arrangement (3) comprises a source bioreactor (5) with a stirrer and
the source bioreactor (5) is a multi-use bioreactor (1) with at least 1000 L production volume of a microbial culture comprising a type of microorganism producing a bioproduct and
the microbial culture of the source bioreactor (5) produces a maximum heat of at least 25,000 W during the source bioprocess and/or
the stirrer of the source bioreactor (5) is driven with a maximum torque of at least 120 Nm,
wherein, according to the target specification data,
the target bioprocess is a continuous microbial bioprocess and
the target bioprocess arrangement (4) comprises a target bioreactor (6) and
the target bioreactor (6) is a single-use bioreactor (2) with a production volume of a microbial culture of at most 50 % of the production volume of the source bioreactor (5) and of at most 800 L,
the microbial culture of the target bioreactor (6) produces a maximum heat of at most 20,000 W during the target bioprocess and/or
the target bioreactor (6) comprises a stirrer driven with a maximum torque of at most 100 Nm,
wherein an average amount of the bioproduct produced per time of performance of the target bioprocess by the target bioreactor (6) is at least 90 %, preferably at least 100 %, of an average amount of the bioproduct produced per time of performance of the source bioprocess by the source bioreactor (5).
